# EUROPEAN PATENT SPECIFICATION

(11) **EP 1 437 096 B1**
(45) Date of publication and mention of the grant of the patent: **31.08.2005**
(21) Application number: 03026740.5
(22) Date of filing: 21.11.2003
(51) Int. Cl.: A61B 17/08, A61F 9/007

(54) **Wound clamp**
Wundklammer
Pince chirurgicale pour fermer les bords d'une plaie

(30) Priority: 07.01.2003 US 337532
(43) Date of publication of application: 14.07.2004
(73) Proprietor: Alcon Inc., 6331 Hunenberg (CH)
(72) Inventor: Charles, Steven T., Germantown Tennessee 38138 (US); Hickingbotham, Dyson W., Stouchsburg Pennsylvania 19567 (US)
(74) Representative: Hanna, Peter William Derek

(56) References cited:
- DE-U- 8 530 432
- DE-U- 8 914 582
- US-A- 5 383 898
- US-B1- 6 197 042
- US-B1- 6 254 624
- DATABASE WPI Section PQ, Week 198945, 20 December 1989 (1989-12-20) Derwent Publications Ltd., London, GB; Class P31, AN 1989-330945 XP002266471 "Device for bringing edges of a wound together" & SU 1 456 109 A (SIBE PHYS TECH INST), 7 February 1989 (1989-02-07)

## Description

### Background of the Invention

Wound clamps are known. An example or a known wound clamp is provided in DE-U-8 914 582 which illustrates a spring-loaded incision clamp which is released by compressing two portions.

This invention relates generally to the field of microsurgery and, more particularly, to ophthalmic microsurgery.

During ophthalmic microsurgery, it is often necessary to remove, dissect, cut, delaminate, coagulate or otherwise manipulate delicate tissues within the eye. Microsurgical tools, such as vitreous cutters, infusion cannulas, micro scissors, micro forceps, illuminated laser probes and other devices are generally used. These devices generally are inserted through one or more surgical incisions in the sclera, a sclerotomy, and different tools may be used during different parts of the surgical procedure. The repeated insertion and removal of these instruments can allow vitreous humor and fluids to escape the eye out through the sclerotomy, increasing the potential for softening of the globe, bleeding, traction on the retina and/or introduction of bacteria into the eye as well as increased healing time.

Therefore, a need continues to exist for a simple, inexpensive clamp for holding the sclerotomy tightly closed during surgery.

### Brief Summary of the Invention

The invention provides a wound clamp according to claim 1.Advantageous embodiments are provided in the dependent claims.

The present invention improves upon the prior art by providing a wound clamp having two substantially identical halves. The clamp halves are biased together using a spring or elastomeric band. Prongs or needles integrally formed with the halves cause the clamp to be engaged firmly on the conjunctival and scleral tissue about the wound. The biasing of the clamp halves toward each other provide for a tight sealing of the wound.

Accordingly, one objective of the present invention is to provide a simple, inexpensive clamp for holding the sclerotomy tightly closed during surgery.

Another objective of the present invention is to provide a clamp have two clamp halves that are biased together using a spring or elastomeric band.

Another objective of the present invention is to maintain alignment of the conjunctiva and scleral incisions if trans-conjunctival surgery is performed.

Still another objective of the present invention is to facilitate introduction of the instruments through the surgical wound.

These and other advantages and objectives of the present invention will become apparent from the detailed description and claims that follow.

### Brief Description of the Drawing

FIG. 1 is a top perspective view of a first embodiment of the wound clamp of the present invention.
FIG. 2 is a bottom perspective view of a first embodiment of the wound clamp of the present invention.
FIG. 3 is a top perspective view of a second embodiment of the wound clamp of the present invention.
FIG. 4 is a bottom perspective view of a second embodiment of the wound clamp of the present invention.

### Detailed Description of the Invention

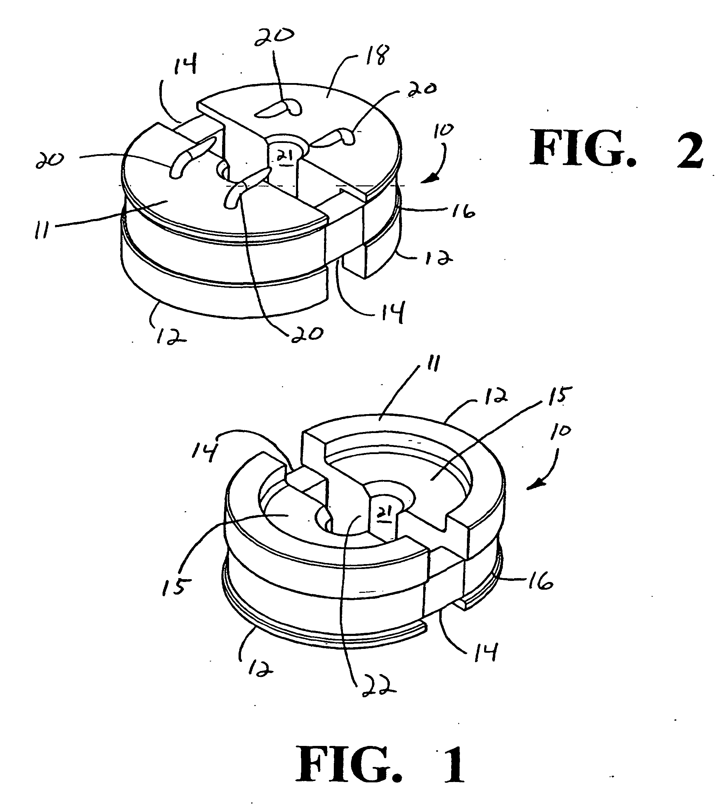

As best seen in FIGS. 1 and 2, in a first embodiment, wound clamp 10 generally consists of body 11 made up of symmetrical halves 12 that are biased together by elastomeric band 14. Band 14 generally fits within groove 16 in halves 12. As best seen in FIG. 2, bottom 18 of halves 12 may contain a plurality of hooks or prongs 20. Halves 12 may be molded or formed from any suitable material, such as thermoplastic, and may contain a lubricious material, such as polytetrafluoroethylene (TEFLON®), and/or a luminous material so as to aid in visualizing clamp 10 in a darkened room. Each of halves 12 contain recess 15 and cut out portions 21 that together define central aperture 22 when halves 12 are biased together. Aperture 22 allows a surgical tool (not shown) to be inserted into a wound when clamp 10 is placed over a surgical site and halves 12 pulled together by band 14. Portions 21 may also be flared, beveled or chamfered to assist in the introduction of the surgical tool into the wound. Recess 15 allows for multiple clamps 10 to nest together during storage, shipping or insertion so as not to damage prongs 20. Band 14 may be made from any suitable elastomeric material, such as silicone rubber. Prongs 20 may be made from any suitable material, such as stainless steel, suture materials such as polypropylene or modified surgical staples and may be integrally formed within halves 12 during the molding process or attached to halves 12 by any suitable process, such as adhesive.

In use, halves 12 of clamp 10 are forced apart by a probe, lance or other suitable tool (not shown) and placed over a surgical incision so that prongs 20 contact the tissue around the incision. The tool is then removed, and the elastomeric nature of band 14 causes halves 12 to be pulled together, thereby forcing prongs 20 in the tissue around the incision and pulling the incision closed and causing clamp 10 to engage firmly on the tissue about the wound. Surgical tools may be inserted into the incision through aperture 22 without causing excessive opening of the incision. In addition, halves 12 may spread apart slightly to enlarge aperture 22 without prongs 20 becoming dislodged from the tissue so as to allow foreign bodies or slightly larger instruments to exit the wound.

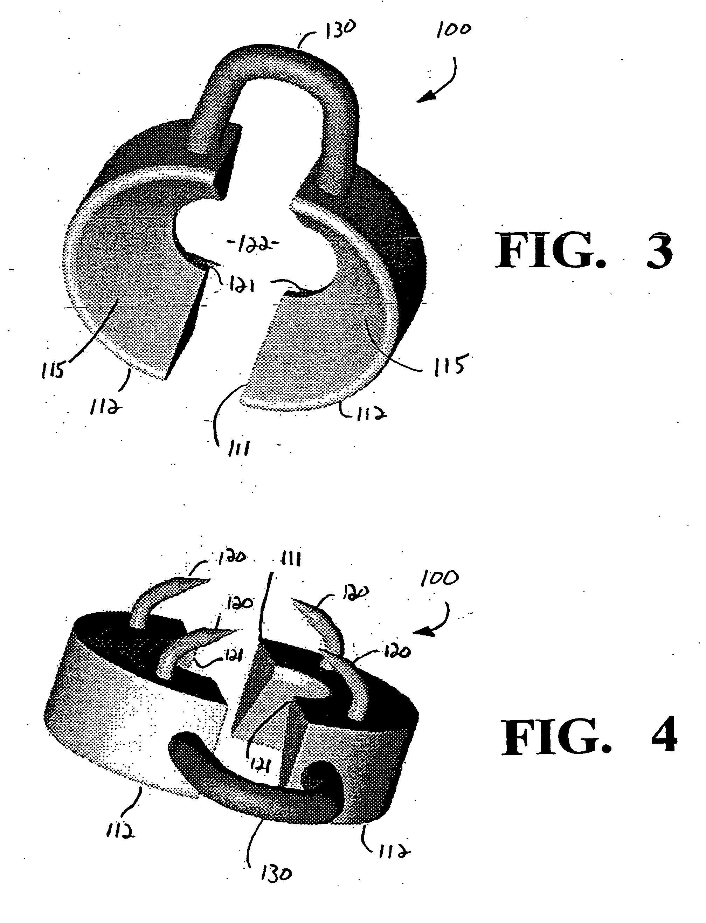

A second embodiment of the present invention, best seen in FIGS. 3 and 4, clamp 100 is of similar construction as clamp 10, body 111 having halves 112, halves 112 having portions 121 that form aperture 122 and prongs 120 and recess 115. Instead of band 14, halves 112 are biased together using spring 130, which may be made from any suitable thermoplastic or metal. Clamp 100 operates in essentially the same manner as clamp 10, described above.

This description is given for purposes of illustration and explanation. It will be apparent to those skilled in the relevant art that changes and modifications may be made to the invention described above without departing from its scope.

## Claims

1. A wound clamp (10, 100), comprising:
a) a body (11,111) having a first half (12,112) and a second half (12,112);
b) a biasing means (14,130) biasing the first half against the second half; and
c) a plurality of prongs (20) associated with both the first half and the second half, for causing the clamp to be engaged firmly on tissue about a wound, in use, and
wherein the first half and the second half (12,112) each contain cut out portions (21,121), **characterised in that** said cut-out portions together form a central aperture (22,122) when the first half and the second half are biased together by said biasing means (14,130), the aperture so defined enabling an insertion of instruments through the aperture to the wound when the clamp is in place.

2. The wound clamp of claim 1, wherein said biasing means comprises an elastomeric band (14).

3. The wound clamp of claim 2, wherein the first half and the second half each have a groove (16) and the elastomeric band (14) fits within said grooves.

4. The wound clamp of claim 1 wherein the biasing means comprises a spring (130).

5. The wound clamp of any one of claims 1 to 4, wherein said body (11,111) comprises a lubricious material.

6. The wound clamp of any one of claims 1 to 5, wherein said body (11,111) comprises a luminous material.

7. The wound clamp of any one of claims 1 to 6, wherein said cut out portions (21,121) are flared, bevelled or chamfered.

8. The wound clamp of any one of claims 1 to 7, wherein said first half and said second half (12,112) each define a recess (15,115) on one side thereof, adapted to allow for multiple clamps (10,100) to nest together.

## Patentansprüche

1. Wundklammer (10, 100), welche umfaßt:
a) einen Körper (11, 111), der eine erste Hälfte (12, 112) und eine zweite Hälfte (12, 112) aufweist;
b) eine Vorspanneinrichtung (14, 130), welche die erste Hälfte gegen die zweite Hälfte vorspannt; und
c) eine Mehrzahl von der ersten Hälfte und der zweiten Hälfte zugeordneten Zinken (20), um zu bewirken, daß die Klammer bei Gebrauch fest an Gewebe um die Wunde angreift und
wobei die erste Hälfte und die zweite Hälfte (12, 112) jeweils Aussparungen (21, 121) umfassen,
**dadurch gekennzeichnet, daß** die Aussparungen, wenn die erste und die zweite Hälfte durch die Vorspanneinrichtung (14, 130) zusammengespannt werden, zusammen eine zentrale Öffnung (22, 122) bilden, wobei, wenn die Klammer angebracht ist, die so definierte Öffnung ein Einführen von Instrumenten zu der Wunde durch die Öffnung ermöglicht.

2. Wundklammer nach Anspruch 1, wobei die Vorspanneinrichtung ein elastomeres Band (14) umfaßt.

3. Wundklammer nach Anspruch 2, wobei die erste Hälfte und die zweite Hälfte jeweils eine Nut (16) aufweisen und das elastomere Band (14) in diese Nuten paßt.

4. Wundklammer nach Anspruch 1, wobei die Vorspanneinrichtung eine Feder (130) umfaßt.

5. Wundklammer nach einem der Ansprüche 1 bis 4, wobei der Körper (11, 111) ein gleitfähiges Material umfaßt.

6. Wundklammer nach einem der Ansprüche 1 bis 5, wobei der Körper (11, 111) ein leuchtendes Material umfaßt.

7. Wundklammer nach einem der Ansprüche 1 bis 6, wobei die Aussparungen (21, 121) sich erweiternd, abgeschrägt oder abgefast sind.

8. Wundklammer nach einem der Ansprüche 1 bis 7, wobei die erste Hälfte und die zweite Hälfte (12, 112) je eine Vertiefung (15, 115) auf einer Seite definieren, die dazu eingerichtet sind zuzulassen, daß mehrere Klammern (10, 100) miteinander verschachtelt sind.

## Revendications

1. Pince pour plaie (10, 100), comportant :
(a) un corps (11, 111) ayant une première moitié (12, 112) et une seconde moitié (12, 112),
(b) des moyens de rappel (14, 130) rappelant la première moitié contre la seconde moitié, et
(c) une pluralité de pointes (20) associées à la première moitié et à la seconde moitié, pour amener la pince à venir en prise de manière ferme avec du tissu autour d'une plaie, en utilisation, et la première moitié et la seconde moitié (12, 112) contenant chacune des parties découpées (21, 121), **caractérisée en ce que** lesdites parties découpées forment ensemble une ouverture centrale (22, 122) lorsque la première moitié et la seconde moitié sont rappelées ensemble par lesdites moyens de rappel (14, 130), l'ouverture ainsi définie permettant une insertion d'instruments à travers l'ouverture jusqu'à la plaie lorsque la pince est en place.

2. Pince pour plaie selon la revendication 1, dans laquelle lesdits moyens de rappel comportent une bande en élastomère (14).

3. Pince pour plaie selon la revendication 2, dans laquelle la première moitié et la seconde moitié ont chacune une gorge (16), et la bande en élastomère (14) s'agence dans lesdites gorges.

4. Pince pour plaie selon la revendication 1, dans laquelle les moyens de rappel comportent un ressort (130).

5. Pince pour plaie selon l'une quelconque des revendications 1 à 4, dans laquelle ledit corps (11, 111) comporte un matériau lubrifiant.

6. Pince pour plaie selon l'une quelconque des revendications 1 à 5, dans laquelle ledit corps (11, 111) comporte un matériau lumineux.

7. Pince pour plaie selon l'une quelconque des revendications 1 à 6, dans laquelle lesdites parties entaillées (21, 121) sont évasées, coniques ou chanfreinées.

8. Pince pour plaie selon l'une quelconque des revendications 1 à 7, dans laquelle ladite première moitié et ladite seconde moitié (12, 112) définissent chacune un évidement (15, 115) sur un côté de celles-ci, adapté pour permettre à de multiples pinces (10, 100) d'être emboîtées ensemble.
